# EUROPEAN PATENT APPLICATION

(11) **EP 4 494 675 A1**
(43) Date of publication of application: **22.01.2025**
(21) Application number: 23186768.0
(22) Date of filing: 20.07.2023
(51) Int. Cl.: A61M 5/20, A61M 5/32, A61M 5/50

(54) **A RELEASE MECHANISM FOR AN INJECTION DEVICE**

(71) Applicant: Ypsomed AG, 3401 Burgdorf (CH)
(72) Inventor: Loser, Peter, 3037 Herrenschwanden (CH); Tschirren, Markus, 3400 Burgdorf (CH); Schrul, Christian, 3400 Burgdorf (CH); Glauser, Oliver, 3008 Bern (CH); Zumstein, Dominik, 3014 Bern (CH); Bosshard, Simon Martin, 3324 Hindelbank (CH)
(74) Representative: Meier Obertüfer, Jürg

(57) **Abstract**

An assembly comprising a housing, a syringe moveable between a needle retracted and a needle inserted position. A plunger rod, axially moveable and rotationally secured to the housing. A release sleeve axially moveable relative to the housing and an injection spring configured to bias the plunger rod towards the distal end of the housing. A first threaded engagement between the release sleeve and the plunger rod configured to rotate the release sleeve when the plunger rod moves towards the distal end of the housing. The release sleeve being rotationally coupled to the housing when the prefilled syringe is axially moved from the needle retracted position towards the needle inserted position and the release sleeve is rotationally decoupled when the syringe has reached the needle inserted position thereby allowing the injection spring to move the plunger rod while rotating the release sleeve.

## Description

### TECHNICAL FIELD

The present invention relates to a release mechanism for an injection device for advancing a plunger rod in a reservoir or to an assembly configured to release a plunger rod to advance a piston in a reservoir.

### BACKGROUND OF THE INVENTION

Injection devices are used for the subcutaneous or transcutaneous delivery of fluid medicaments to the patient. The injection device may be an infusion pump, an injection pen, an auto injector, a patch injector or an autopen. The injection device may be a reusable or disposable device. The fluid medicament is contained in a reservoir and delivered through an outlet of the reservoir by reducing the volume available for the medicament. The wall of the reservoir may be constructed from a flexible material and fluid contained within reservoir is delivered through the outlet by compressing the flexible wall. Alternatively, the reservoir may be constructed from a rigid material such as glass or a rigid plastic and a moveable plunger or piston closes the reservoir at a location opposite from the outlet. The injection device includes a delivery mechanism for advancing the plunger in the reservoir thereby reducing the volume available for the medicament and expelling fluid through the outlet. A needle may be connected or connectable to the outlet. For a prefilled syringe (PFS), a stacked hollow needle is directly and fixedly connected to the outlet of a glass barrel. The needle of the prefilled syringe may penetrate the skin of a patient during use of the injection device. The outlet of the reservoir may be connectable to a needle, for example using a luer-lock connection. The outlet of the reservoir may be connected or connectable to a fluid path including a tubing and ending in a hollow needle or cannula that is configured for skin insertion.

The injection device may include an apparatus for covering the needle before and after the injection thereby reducing the risk of undesired needle sticking during use of the injection device and/or accommodating the injection device to patients suffering from needle phobia. Such an apparatus may include a needle cover sleeve that may move with respect to a housing of the injection device against the bias of a cover sleeve spring ensuring that the tip of the needle is covered before and after the injection by the needle cover and during the injection the needle is inserted into the tissue.

The advancement of the plunger in the rigid reservoir or the compression of the walls of the flexible reservoir requires a power package delivering the energy required for advancing the plunger or compressing the reservoir. For a rigid reservoir, the plunger may be advanced by a delivery mechanism including a plunger rod. The power package may include a biasing member such as an injection spring and the injection spring is retained in a compressed state until medicament delivery is started. The injection may be started by pressing an activation button on the injection device or the injection may be started by pressing the needle cover sleeve against the skin of the patient. The activation button requires a spring member biasing the button to the non-activated position and the needle cover sleeve is moved against the bias of a needle cover sleeve spring such that the needle cover sleeve returns back to its original position after the injection for covering the needle tip.

US20210361881 A1 discloses an auto injector including a clutch having a fixed axial position within the body and being coupled a lockout shroud such that a movement of the lockout shroud rotates the clutch to release a driver system. The lockout shroud is moved against the bias of a spring and the driver system advances a plunger rod using an injection spring.

WO16169756 A1 discloses an auto injector with a plunger rod that may be advanced by a drive spring. The auto injector is activated by an activator (cover sleeve) that is axially moved against a delivery member guard spring thereby rotating a rotator to release the drive spring for delivery of a medicament from a prefilled syringe.

There may be a need for injection devices with assemblies providing a release mechanism using less parts thereby reducing the environmental impact of the devices. There may be a need for injection devices with assemblies providing an alternative and reliable release mechanism. There may be a need for injection devices with release mechanisms that may be paused during an injection or may be terminated before completely emptying the medicament reservoir.

It is an object of the present invention to overcome the disadvantages of the prior art. This objective is solved by the independent claim and specific variants are disclosed in the dependent claims, the description and the figures.

### DEFINITIONS

The term "medicament" or "medication" includes any flowable medical formulation suitable for controlled administration through a means such as, for example, a cannula or a hollow needle and includes a liquid, a solution, a gel or a fine suspension containing one or more medical active ingredients. A medicament can be a composition including a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The distal end or distal direction is defined by the direction of the hollow needle configured to penetrate the skin of the patient. For a medicament delivery device such as an injection pen or auto injector this may be the injection needle and the end of the pen or auto injector holding the needle or being configured to hold the needle is the distal end. For an infusion device the distal end and the distal direction is towards the needle configured to penetrate the skin of the patient, which may be along the axis of the device or tilted or perpendicular to the axis of the device. The distal direction in an infusion device represents the direction in which the medicament flows towards the insertion needle. The proximal direction or end is opposite to the distal direction or end.

In the claims, the word "comprising" does not exclude other elements or steps, and the indefinite article "a" or "an" does not exclude a plurality. For example "an injection spring" does not exclude the fact that there may be two injection springs that functionally or structurally fulfill the purpose of "an injection spring". The mere fact that certain elements or steps are recited in distinct claims shall not preclude the existence of further meaningful combinations of these elements or steps.

### GENERAL DESCRIPTION OF THE INVENTION

The present disclosure may relate to a release mechanism for an injection device for advancing a plunger rod in a reservoir or to an assembly configured to release a plunger rod to advance a piston in a reservoir or to a release apparatus for an injection device. The present disclosure may relate to an injection device including the release mechanism or the assembly configured to release an injection from the injection device. The injection device may be a fixed dose or a variable dose injection device. The injection device may be an auto injector, an autopen or a patch injection device. The injection device may be a reusable or a disposable device.

The injection device includes a power package for advancing a plunger rod. The plunger rod in the auto injector may be spring driven, electrically driven, pneumatic driven, driven by a compressed gas or hydraulic driven. The plunger rod in the autopen may be spring driven or may be electromechanically driven. The plunger rod in the patch injection device may be spring driven, electrically driven or electromechanically driven. The plunger rod of the injection device may be driven by a hybrid mechanism based on a spring and an electric drive.

The release mechanism, or the assembly for the injection device includes the plunger rod that is biased in the distal direction by the power package for advancing a piston or plunger in the reservoir thereby expelling medicament from the injection device. The bias on the plunger rod is converted into a torque on a release sleeve. The torque may be applied by a threaded engagement between the release sleeve and the plunger rod or by an electromagnetic force applied between the housing and the release sleeve or via a link motion system between the plunger rod and the release sleeve or between the release sleeve and the housing. The release sleeve is releasably coupled or secured to the housing and rationally locked to the housing thereby preventing rotation against the torque applied and thereby preventing axial advancement of the plunger rod. The coupling between the release sleeve and the housing may be released, for example by axially moving the release sleeve or by pushing a button on the housing or by releasing an electromagnetic coupling such that the release sleeve may rotate due to the bias applied by plunger rod. The coupling may be a splined type of coupling between an axial key and a complementary groove and upon ending the interaction between the key and the groove the release sleeve may rotate with respect to the housing. The key and complementary groove may be present on the housing and the release sleeve. Alternatively, a dog type of coupling may be used whereby dog teeth may be disengaged from the housing when the release sleeve is axially moved. The coupling may be directly positioned between the housing and the release sleeve or indirectly between the release sleeve and a housing part or an intermediate part.

The assembly for the release mechanism includes a housing, a syringe moveable between a needle retracted and a needle inserted position and a plunger rod, axially moveable and rotationally secured to the housing. The assembly further includes a release sleeve axially moveable relative to the housing and an injection spring configured to bias the plunger rod towards the distal end of the housing. A first threaded engagement may be provided between the release sleeve and the plunger rod configured to rotate the release sleeve when the plunger rod moves towards the distal end of the housing. The release sleeve being rotationally coupled to the housing when the prefilled syringe is axially moved from the needle retracted position towards the needle inserted position and the release sleeve is rotationally decoupled when the syringe has reached the needle inserted position thereby allowing the injection spring to move the plunger rod while rotating the release sleeve.

The assembly for the injection device includes a housing defining a longitudinal axis. The assembly further includes a reservoir or syringe with an attached needle, or a reservoir configured for attaching a needle or for attaching a fluid conduit to the reservoir. The housing may be a single part housing or may be assembled from multiple parts. The housing may be provided with inserts for holding other parts of the assembly such as, for example the reservoir or syringe or the plunger rod or an injection or actuation button. The reservoir or prefilled syringe may be located within the housing or within a housing part such as, for example, a reservoir holder or a syringe holder.

The syringe or reservoir may be a prefilled syringe (PFS) and the medicament may be a liquid or a solid that may be dissolved in a fluid matrix or is suspended in a fluid matrix. The syringe or reservoir may be axially moveable with respect to the housing along the longitudinal axis between a needle retracted position and a needle inserted position defining a needle insertion distance. The movement may be a reversible movement from the needle retracted position to the needle inserted position and back from the needle inserted position to the needle retracted position. The movement may be repeated. The reservoir or syringe may be moveable towards the needle inserted position or towards the needle retracted position. The movement may be a manual movement provided by a user pushing on a push button. The movement may be initiated by a biasing member biasing the reservoir or syringe towards the needle inserted and/or towards the needle retracted position.

The assembly includes the plunger rod that may be axially moveable parallel or perpendicular to the longitudinal axis of the housing. The plunger rod may be rotationally secured to the housing, or to a housing part or to an insert for the housing and may be configured to engage the piston or plunger in the syringe or reservoir for expelling medicament from the injection device. The piston may be advanced in the syringe or reservoir towards an outlet thereby reducing the volume available for the medicament in the reservoir as the medicament is expelled via the outlet. The outlet of the reservoir may be directly coupled to the needle or the outlet is coupled or configured to be coupled to a fluid conduit. The fluid conduit may include a tubing ending in a hollow needle or cannula adapted for insertion into the skin of a patient. The housing may include an insert with a passage that is splined to an outer surface of the plunger rod preventing relative rotation between the plunger rod and the insert. The insert may be secured or releasably secured to the housing thereby preventing rotation between the plunger rod and the housing.

Alternatively, the plunger rod is rotatable and axially moveable relative to the housing, for example along a helical path while moving from the needle retracted to the needle inserted position. A rotatable flange may be located at the distal end of the plunger rod. Optionally the housing comprises an internal threading engaging an outside threading on the piston rod for guiding and axially translating the plunger rod during an injection. The housing may include an insert provided with the internal threading.

The assembly includes the release sleeve that may be axially moveable relative to the housing.

The release sleeve may be rotationally coupled or configured to be coupled to the housing. The release sleeve may be coaxially arranged around the plunger rod. The release sleeve may directly surround the plunger or indirectly surround the plunger rod via another part located between the release sleeve and the plunger rod. The release sleeve may axially move relative to the housing and towards the distal end of the housing from the needle retracted position to the needle inserted position over the needle insertion distance. The release sleeve may move back from the needle inserted position towards the needle retracted position. The release sleeve may be decoupled from the housing during movement from the needle retracted position to the needle inserted position. The release sleeve may be coupled to the housing when moving from the needle inserted position to the needle retracted position.

The assembly includes a power package that is arranged between the plunger rod and the release sleeve whereby the power package is configured to bias the plunger rod towards the distal end of the housing. The power package may include an injection spring. An alternative power package may be used based on a compressed gas or liquid for a pneumatic or hydraulic drive. The bias may be provided by an electric drive. The power package may be in a compressed or tensioned state before injection and may be decompressed during the injection. The injection spring may be a compression spring or a torsional spring and the distal end of the injection spring may abut an end surface of the plunger rod. The injection spring may be arranged inside the plunger rod or may surround the plunger rod. The injection spring may be operatively arranged between the plunger rod and the release sleeve. The injection spring may bias directly the release sleeve or bias the release sleeve via ta least another part such as, for example a spring sleeve and or a combination of the spring sleeve and a clutch

The injection spring or power package may bias the release sleeve and/or a syringe holder towards the proximal end of the housing. The injection spring or power package may directly or indirectly bias other parts towards the proximal end of the housing such as, for example, an injection button.

The assembly may include a first threaded engagement between the release sleeve and the plunger rod and the threaded engagement may be configured to rotate the release sleeve when the plunger rod moves towards the distal end of the housing. A threaded engagement is defined as a thread segment engaging a groove, or a cam engaging a follower. The thread segment may be a single or multiple thread segment. The threaded engagement may also be called a cam control or link-motion system.

The release sleeve is rotationally coupled to the housing when the prefilled syringe is axially moved from the needle retracted position towards the needle inserted position or from the needle inserted position back to the needle retracted position. The rotational coupling may be a key-groove engagement or a dog type clutch coupling. The rotational coupling may be a splined engagement oriented parallel to the longitudinal axis.

The release sleeve may be rotationally decoupled or unlocked from the housing when the prefilled syringe has reached the needle inserted position thereby allowing the injection spring to release its spring force and to move the plunger rod towards the distal end of the housing while rotating the release sleeve via the first threaded engagement. The plunger rod advances the piston or plunger in the reservoir for expelling medicament from the reservoir. The decoupling may be due to the disengagement of the splined engagement between the release sleeve and the housing or due to the unlocking of a form fit engagement to the housing, for example by pressing an actuation button that may be different from an injection button.

The assembly may further include a reservoir or syringe holder for holding the syringe or reservoir. The syringe holder or reservoir holder may be axially moveable relative to the housing over the needle insertion distance from the needle retracted position to the needle inserted position, preferably at the start of the injection. The syringe holder or reservoir holder may be axially moveable relative to the housing back from the needle inserted position to the needle retracted position, preferably at the end of the injection or when the injection has been completed or temporarily paused or prematurely stopped.

The syringe holder may hold the syringe, for example support and hold a shoulder section located towards the distal end of the syringe and/or may support a finger flange located at the proximal end of the syringe. The syringe holder may include elastic shock absorbers located between the syringe and the syringe holder.

The syringe holder may be keyed to the housing, for example in a splined (linear) engagement. The linear engagement may be parallel or perpendicular to the longitudinal axis. Alternatively there may be a link-motion between the syringe holder and the housing for a combined linear and rotational movement.

Axial movement of the syringe holder with respect to the housing may be prevented by a cap or plug located the distal end of the housing. The cap may abut the syringe holder. Removal of the cap may ensure that the syringe holder may be axially moved relative to the housing. The cap is preferably removed before starting the injection thereby allowing the movement of the syringe holder towards the needle inserted position. A threshold force may have to be overcome before starting the movement to disconnect a releasable connection between the housing and the syringe holder.

The cap or plug may include a needle shield remover for removing a needle shield located at the distal end of the reservoir or syringe preventing contamination of the content within the syringe or the reservoir and providing a sterile barrier. The assembly of the cap and the needle shield may be called a cap remover. Removal of the cap may unlock or unblock or release an engagement between the housing and the syringe holder, for example a snap fit connection including an elastic arm or a latching mechanism and flexing of the arm is prevented by the cap remover. The snap fit connection or the latching mechanism may provide for a threshold force that must be overcome before starting an injection. Once the cap has been removed, the arm can flex and the syringe holder may be moved relative to the housing. Relative movement between the syringe holder and housing may be allowed after cap removal and/or after releasing the latching mechanism. The cap and/or the mechanism providing the threshold force may prevent premature or unintended activation of the injection device provided with the assembly.

The syringe holder in the assembly is axially coupled to the release sleeve and the release sleeve is configured to be rotatable relative to the syringe holder. The axial coupling may be due to a key/groove engagement that may be oriented perpendicular to the longitudinal axis.

Axially moving the release sleeve may also axially move the syringe holder, or axially moving the syringe holder may axially move the release sleeve. The combination of the release sleeve and the syringe holder is axially moveable from the needle retracted to the needle inserted position and from the needle inserted position back to the needle retracted position. The release sleeve may axially move together with the syringe holder over the needle insertion distance without rotation or the release sleeve may rotate during the axial movement. Alternatively, the release sleeve may start rotating once the syringe holder has reached the needle inserted position while the syringe holder is prevented from rotation.

The assembly may further include a spring sleeve that is rotationally coupled or rotationally secured to the housing and axially moveable relative to the housing over the needle insertion distance.

The spring sleeve may be splined or keyed to the housing. Preferably the spring sleeve remains rotationally coupled and axially moveable relative to the housing whether moved over the needle insertion distance or when the syringe holder has reached the needle inserted position.

The syringe or reservoir is preferably located axially between the syringe holder and the spring sleeve. A spring element may be part of the spring sleeve and may be located at the distal end of the spring sleeve. The spring element may be located between the spring sleeve and the proximal end of the syringe or reservoir. The spring element may abut and bias the syringe or reservoir towards the distal direction, for example into the syringe holder. The spring element may abut a proximal end, for example a finger flange provided at the proximal end of the syringe or reservoir.

The power package or the injection spring may bias the spring sleeve or a proximal end wall of the spring sleeve towards the proximal end of the housing.

The spring sleeve for the assembly may be coaxially arranged between the plunger rod and the release sleeve. A proximal end of the injection spring may act on the spring sleeve and/or the release sleeve thereby providing a bias in the proximal direction on the spring sleeve and/or the release sleeve. The distal end of the injection spring may act on the plunger rod thereby biasing the plunger rod in the distal direction. The spring sleeve may directly surround the plunger rod. The release sleeve may coaxially surround the spring sleeve.

The plunger rod may include at least one protrusion oriented perpendicular to the longitudinal axis or radially outwards to the axis for engaging a guide slot in the spring sleeve, the guide slot may be oriented parallel to the longitudinal axis, thereby axially guiding the plunger rod. The spring sleeve may be axially moveable relative to the housing whereas the piston rod is axially moveable and rotationally secured to the spring sleeve. The piston rod is rotationally secured with respect to the housing via the spring sleeve.

The assembly of the syringe holder, the syringe or reservoir, the plunger rod, the injection spring, the spring sleeve and the release sleeve may be axially moveable along the longitudinal axis over the needle insertion distance by pressing, for example, a push button.

The at least one protrusion on the outside surface of the plunger rod may engage the first thread segment or groove on the inside of the release sleeve, the first thread segment or groove having a first pitch whereby the first thread segment and the protrusion may provide the first threaded engagement. During axial advancement of the plunger rod without rotation, the release sleeve may rotate due to the first threaded engagement.

As an alternative, the first thread segment may be located on the outside surface plunger rod and the protrusion on the release sleeve thereby providing the first threaded engagement. In this case the plunger rod may rotate during advancement and may be guided by a helical guide slot present in the spring sleeve.

The assembly may further include the injection button axially guided by the housing and moveable between a button retracted position and a button actuated position defining a button actuation distance. The injection button may be prevented from rotation with respect to the housing. The injection button may be a sleeve or a grip or a push button. The injection button may be provided as a push button located at the proximal end of the housing or as a sleeve or grip that at least partially surrounds the housing. The user may hold the housing with his fingers while pushing the push button at the proximal end with his thumb. Alternatively, the user grips the whole sleeve or grip with his hand while pushing the housing against the skin for axially moving the sleeve with respect to the housing for starting the injection.

In one embodiment, the button actuation distance is greater than the needle insertion distance. The injection button may move the assembly of the syringe holder with the syringe or reservoir, the plunger rod, spring sleeve and release sleeve in the distal direction and the assembly may be pressed against the bias provided by the power package in the proximal direction, for example against the bias provided by the injection spring. The difference between the button actuation distance and the needle insertion distance may be used for providing a gearing arrangement facilitating the advancement of the assembly and/or additional compression of the spring. The gearing arrangement may gear down the axial movement of the injection button to the assembly of the syringe holder with the syringe or reservoir, the plunger rod, spring sleeve and release sleeve. The gearing down may facilitate pressing the assembly against the force provided by the injection spring.

In another embodiment, the button actuation distance is smaller than the needle insertion distance and the gearing arrangement may gear up the movement of the injection button to the assembly of the syringe holder with the syringe or reservoir, the plunger rod, spring sleeve and release sleeve.

The assembly may further include a clutch sleeve. The clutch sleeve may be coaxially arranged between the injection button and the release sleeve. The above described gearing arrangement may further include the clutch sleeve. The clutch sleeve may be at least partially inside the housing or the clutch sleeve may at least partially surround the housing

The injection button may be the outermost part of the assembly and may at least partially surround the housing and/or surround the clutch sleeve.

The clutch sleeve may include a proximal end wall for abutting the injection button. An axial force provided on the injection button and directed in the distal direction may be transferred to the clutch sleeve via the proximal end wall. The clutch sleeve may be formed as a cylindrical sleeve closed by the end wall. The cylindrical sleeve and end wall may provide a bore and the cylindrical sleeve may at least partially surround the release sleeve. The cylindrical wall of the clutch sleeve extending into the distal direction may be located inside the housing or surround the housing. The cylindrical wall of the clutch sleeve may be engaged with the housing in a splined or a threaded engagement.

The clutch sleeve may follow the axial movement of the injection button due to the abutment between the end wall and the injection button. The injection button may be closed by a proximal wall that may abut the end wall of the clutch sleeve.

The clutch sleeve may rotate with respect to the injection button as the button is axially moved over the button actuation distance. A pivot bearing may be provided between the proximal wall of the push button and the proximal end wall of the clutch sleeve to reduce the frictional losses when the push button is moved towards the button actuated position thereby rotating the clutch sleeve. The clutch sleeve and/or the injection button may be made from plastic materials adapted to reduce frictional losses. The plastic material may include an additive such as silicone oil, or polytetrafluoroethylene (PTFE) to reduce friction. Optionally a friction reducing agent, such as silicone oil may be applied to the surfaces.

The clutch sleeve may be threaded engaged with the housing via a third threaded engagement such that the clutch sleeve rotates when the dose button axially moves from the button retracted position to the button actuated position.

The third threaded engagement may include a plurality of helical grooves on an inside surface of the housing engaging a plurality of helical thread segments that protrude radially outwards from the clutch sleeve, or from the outside surface of the cylindrical sleeve. Alternatively, the third threading is provided as helical thread segments that protrude from the housing inwards towards the longitudinal axis engaging helical grooves present on the outside surface of the clutch sleeve or outside surface of the cylindrical sleeve.

The third threaded engagement may be located at the proximal and or at the distal end of the clutch sleeve.

The clutch sleeve may be threaded engaged with the release sleeve via a second threaded engagement and wherein the second and third threaded engagements have the same hand. The second threaded engagement may be provided as a helical thread segment or as a plurality of helical thread segments that protrude radially outwards from the release sleeve and which are configured to engage a matching helical groove or a plurality of helical grooves located on an inside surface of the clutch sleeve. Alternatively, the second threading may be provided as a helical groove or a plurality of helical grooves present on the outside surface of the release sleeve engaging a helical thread or plurality of thread segments that protrude radially inward from an inside surface of the clutch sleeve, preferably from an inside surface of the cylindrical wall.

The second and/or third threaded engagements may provide for the gearing arrangement, gearing down or gearing up the axial movement of the injection button (over the button actuation distance) to the axial movement of the assembly including the syringe holder and syringe or reservoir being moved over the needle insertion distance.

The second and third threaded engagements may be provided as left handed threading or right handed threading. The second and third threaded engagements may be provided as a single threading, or as a double threading, or as a triple threading.

The second and third threaded engagements preferably have a different hand compared to the first threaded engagement. The second and third threaded engagements may be right handed whereas the first threaded engagement may be left handed or vice versa, the second and third threaded engagements may be left handed whereas the first threaded engagement may be right handed.

The three threaded engagements may have the same pitch, whereby the pitch is defined as the angle between the threaded segments and the longitudinal axis. The three threaded engagements may each have a different pitch. The first threaded engagement may have the lowest pitch, the lowest angle between the thread segments and the longitudinal axis. The second threaded engagement may have the highest pitch. The pitch of the third threaded engagement may be between the pitch of the first and the third threaded engagements.

In an embodiment, the clutch sleeve may include a protrusion oriented along the longitudinal axis and guided through an opening in the release sleeve and wherein the protrusion abuts a distal end of the spring sleeve.

The protrusion may extend from the end wall of the clutch sleeve in the distal direction, the protrusion may follow the axial movement of the push button due to the abutment between the end walls and/or due to the pivot bearing. The protrusion may extend through the opening in the release sleeve and abut an end wall of the spring sleeve such that axial forces may be transmitted from the push button to the spring sleeve, for example distally directed forces on the push button at the start of the injection. Axial forces from the power package directed in the proximal direction may be transmitted from the spring sleeve to the clutch sleeve via the protrusion. The end wall of the release sleeve provided with the opening may axially move relative to the end wall of the clutch sleeve. The end wall of the release sleeve may be positioned axially between the end wall of the clutch sleeve and the end wall of the spring sleeve.

The clutch sleeve may rotate around the release sleeve as the injection button axially moves towards the button actuated position and the release sleeve may axially move towards the clutch sleeve due to the second and third threaded engagements thereby compressing the injection spring by a distance equal to the difference between the button actuation distance and the needle insertion distance.

During the axial movement of the injection button, the clutch sleeve may be forced to axially move as well due to the abutment with the end wall of the injection button. The clutch sleeve is threaded engaged with the housing via the third threaded engagement such that clutch sleeve may rotate while axially advancing in the distal direction. The release sleeve is axially guided by the housing and is advanced by the clutch sleeve via the second threaded engagement. As the clutch sleeve rotates around the non-rotating release sleeve, the release sleeve is axially moved preferably towards the clutch sleeve via the second threaded engagement thereby closing a first axial gap between the end wall of the release sleeve and the end wall of the clutch sleeve. The release sleeve, and therewith the syringe holder including the syringe or reservoir are moved about a distance that is reduced by the distance for closing the first axial gap. The injection button and the clutch sleeve may be moved over the button actuation distance while the release sleeve and the syringe holder may be moved over the button actuation distance reduced by the distance for the first axial gap. The needle insertion distance may be calculated as the button actuation distance minus the first axial gap.

The axial force provided by the injection spring (or power package) that is directed in the proximal direction and which is guided to the injection button is geared down using the second and third threaded engagements between the release sleeve and the clutch sleeve, respectively between the clutch sleeve and the housing. The axial force of the injection spring may be directed in the distal direction to the plunger rod and in the proximal direction to the spring sleeve and via the protrusion on the end wall to the clutch sleeve. The force that the user may require for pushing the injection button against the bias provided by the injection spring may be geared down as the injection spring may provide a high injection force on the plunger rod and on the injection button if no gearing arrangement is provided. The gearing arrangement may be beneficial for the user attempting to push the injection button.

When the user pushes the injection button over the button actuation distance for starting the injection, then a second axial gap between the plunger rod and the spring sleeve may be closed thereby further compressing the injection spring. The first axial gap between the clutch sleeve and the release sleeve is closed as the release sleeve is moved in the proximal direction relative to the clutch sleeve. The release sleeve moves the syringe holder (axially coupled to the release sleeve) in the proximal direction relative to the clutch sleeve whereas the spring sleeve is held in an axially fixed position as the spring sleeve abuts the protrusion on the end wall of the clutch sleeve. The second axial gap between the spring sleeve and the release sleeve is closed thereby compressing the injection spring by a distance that may be equal to the second axial gap. The axial distance for the first and second axial gaps may be equal.

The additional compression of the injection spring at the start of the injection may be used for retracting the needle from the needle inserted position to the needle retracted position. The movement back towards the needle retracted position may be initiated during an injection for pausing an injection that has been ongoing without fully emptying the syringe, or may be initiated after emptying the syringe and the spring force may be used for activation a lockout mechanism that prevents re-use or repeated use of the device.

The release sleeve may be decoupled from the housing, for example the splined engagement or the dog type of coupling is uncoupled once the syringe holder and the release sleeve have reached the needle inserted position. Once the syringe holder has reached the needle inserted position, the third threaded engagement between the housing and the clutch sleeve may be released, for example disengaged. The thread segment on the clutch sleeve may not engage the complementary threading in the housing anymore and the clutch sleeve is free to rotate without axial movement and free to rotate together with the release sleeve as the release sleeve is forced to rotate due to the first threaded engagement with the plunger rod.

The release sleeve may be first decoupled from the housing (for example the splined engagement or dog clutch) before decoupling the third threaded engagement between the clutch sleeve and the housing or, vice versa, the third threaded engagement between the housing and the clutch sleeve is released before opening the clutch between the release sleeve and the housing.

The clutch sleeve may co-rotate with the release sleeve due to the second threaded engagement forcing the clutch sleeve to co-rotate with the with the release sleeve as the clutch sleeve is not coupled to the housing via the third threaded engagement.

Pause function: The user may partially release the injection button during an injection e.g. when the plunger rod has not reached the final position yet, and move the injection button from the actuated position towards the button retracted position and hold the button at an intermediate axial position. The additional compression provided by the injection spring while starting the injection will force the spring sleeve in the proximal direction when pausing the injection. The spring sleeve moves the clutch sleeve in the proximal direction as a part, for example a protrusion, of the clutch sleeve directly abuts the spring sleeve. The clutch sleeve drives the release sleeve in the proximal direction due to the second threaded engagement. The coupling between the release sleeve and the housing is moved back into the coupled position thereby preventing rotation of the release sleeve thereby stopping the injection as the first threaded engagement intends to rotate the release sleeve under the bias of the spring force on the plunger rod and this rotation of the release sleeve is prevented. The clutch sleeve is moved in the proximal direction and the third threaded engagement is reestablished such that the clutch sleeve rotates with respect to the housing and around the non-rotating release sleeve. The gearing engagement is re-established and the first axial gap is opened again and the additional compression of the injection spring is released as the clutch sleeve and therewith the injection button are moved in the proximal direction.

Once the user pushes again on the injection button towards the button actuated position, the injection is restarted again and the plunger rod starts moving from an intermediate position towards an end position for emptying the syringe.

Premature stop: The user may fully release the injection button during an injection, e.g. applies no pressure on the actuation button and the injection button is moved back to the button retracted position and the push button may be prevented from further actuation. The injection button is moved back due to the additional spring force applied during the start of the injection as the release sleeve is rotationally coupled to the housing and the third threaded engagement for the clutch sleeve is reestablished. A motion link system or lockout mechanism between the housing and the injection button or between the syringe holder and the housing may be activated such that the injection button cannot be pressed again. The injection is halted or blocked and the syringe may contain a residual amount of medication.

Final stop: Once the plunger rod has moved to the final position of the syringe, the user may fully release the injection button which is moved back to the proximal position or non-actuated position and the motion link system axially locks the injection button or the syringe holder to the housing preventing further injections or reuse of the device.

While the invention has been described in detail in the drawings below and foregoing general description, such description is to be considered illustrative or exemplary and not restrictive. Variations to the disclosed embodiments can be understood and effected by those skilled in the art and practicing the claimed invention, from a study of the drawings, the disclosure, and the appended claims.

### LEGENDS TO THE FIGURES

- Figure 1a:: Auto injection device, initial state,
- Figure 1b:: Auto injection device with removed cap or needle shield remover,
- Figure 1c:: Auto injection device, injection button moved into the actuated position over the button actuation distance, prefilled syringe has moved over the needle insertion distance,
- Figure 1d:: Auto injection device, syringe emptied,
- Figure 1e:: Auto injection device, injection completed, injection button and syringe moved back in their retracted positions,
- Figure 2:: Exploded view of the auto injection device,
- Figure 3a:: Longitudinal section for the auto injection device, initial state,
- Figure 3b:: Longitudinal section for the auto injection device, initial state. Section in a plane perpendicular to Figure 3a,
- Figure 4:: Assembly of the plunger rod, the spring sleeve, the release sleeve and clutch sleeve,
- Figure 5a:: Longitudinal section for the auto injection device, needle shield remover removed,
- Figure 5b:: Longitudinal section for the auto injection device, section in a plane perpendicular to Figure 5a,
- Figure 6a:: Longitudinal section for the auto injection device, injection button moved towards the actuated position and the prefilled syringe has moved towards the needle inserted position,
- Figure 6b:: Longitudinal section for the auto injection device, section in a plane perpendicular to Figure 6a,
- Figure 7a:: Longitudinal section for the auto injection device, injection button moved into the actuated position and the prefilled syringe has moved into the needle inserted position,
- Figure 7b:: Longitudinal section for the auto injection device, section in a plane perpendicular to Figure 7a,
- Figure 8a:: Longitudinal section for the auto injection device, injection button moved into the actuated position and the plunger rod has moved in the prefilled syringe towards the needle,
- Figure 8b:: Longitudinal section for the auto injection device, section in a plane perpendicular to Figure 8a,
- Figure 9a:: Longitudinal section for the auto injection device, injection button moved into the actuated position and the plunger rod has moved in the prefilled syringe to the final position. The syringe has been completely emptied,
- Figure 9b:: Longitudinal section for the auto injection device, section in a plane perpendicular to Figure 9a,
- Figure 10a:: Longitudinal section for the auto injection device, final state with injection button moved back into the button retracted position,
- Figure 10b:: Longitudinal section for the auto injection device, section in a plane perpendicular to Figure 10a,
- Figure 11a:: Lockout mechanism for the injection device between the syringe holder and the housing; initial state,
- Figure 11b:: Lockout mechanism for the injection device between the syringe holder and the housing; syringe holder moved towards the needle inserted position,
- Figure 11c:: Lockout mechanism for the injection device between the syringe holder and the housing; syringe holder in needle inserted position,
- Figure 11d:: Lockout mechanism for the injection device between the syringe holder and the housing; syringe holder moved from the in needle inserted position towards the needle retracted position,
- Figure 11e:: Lockout mechanism for the injection device between the syringe holder and the housing; syringe holder moved into the needle retracted position, the lockout mechanism is in a locked state.

### DETAILED DESCRIPTION OF THE FIGURES

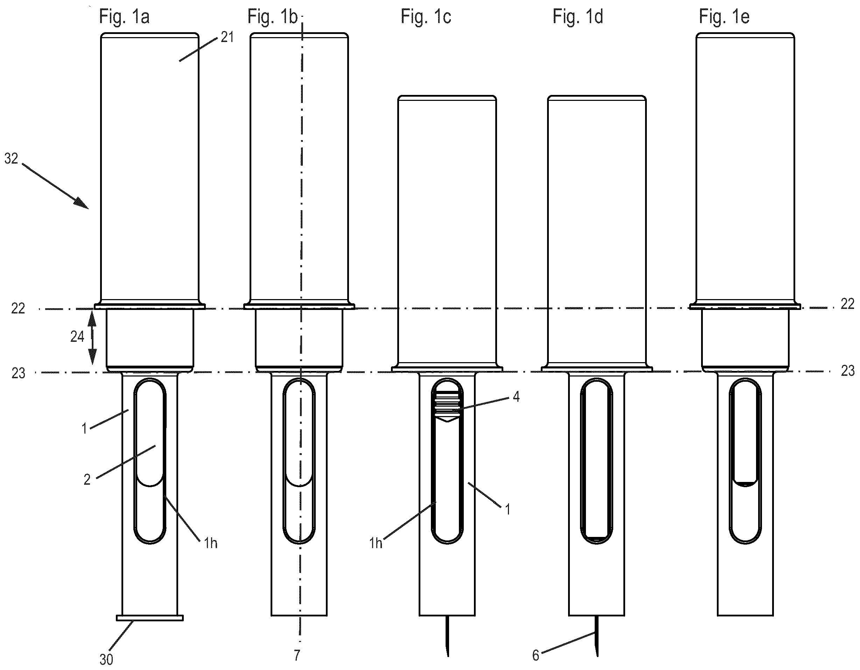

Figures 1a to 1e present an auto injector 32 including an injection button 21, a housing 1 including a viewing window 1h and a prefilled syringe 2, the contents of which can be viewed through the viewing window 1h. The housing 1 is sleeve shaped defining a longitudinal axis 7 and the dimensions of the housing 1 perpendicular to the longitudinal axis may vary, the distal section surrounding the prefilled syringe 2 may have a smaller outer dimension compared to the proximal section enclosing a delivery mechanism. Alternatively, the housing may have a constant outer dimension along the longitudinal axis. The housing may have a circular, rectangular, elliptical or triangular cross section. The injection button 21 may be sleeve shaped and at least partially surround the housing 1. The sleeve may be gripped by the user for starting medicament delivery. As an alternative, the injection button 21 is located at the proximal end of the housing and is at least partially surrounded by the housing 1. In an initial state, prior to medicament delivery, the housing 1 is closed at the distal end by a needle shield remover 30 intended to remove a needle shield closing the prefilled syringe. In the initial state (Figure 1a), the injection button 21 may not be moved with respect to the housing 1. The presence of the needle shield remover 30 may directly or indirectly block the relative movement between the injection button 21 and the housing 1. The user may remove the needle shield remover 30 from the distal end of auto injector 32, thereby removing the needle shield from the prefilled syringe (Figure 1b). Subsequently, the injection button 21 may be moved axially with respect to the housing 1 from a button retracted position 22 (Figures 1a and 1b) to a button actuated position 23 (Figure 1c) defining a button activation distance 24. The injection button 21 is operatively coupled to the prefilled syringe 2, for example operatively coupled to a syringe holder holding the prefilled syringe, such that prefilled syringe and the needle 6 is moved along the longitudinal axis 7 from a needle retracted position to a needle inserted position defining a needle insertion distance (Figure 1c). The needle insertion distance is smaller than the button actuation distance such that the movement of the injection button is geared down by the operative coupling between the injection button and the syringe holder. Advancement of the prefilled syringe 2 ensures that the needle 6 projects from the distal end of the housing for skin insertion. A piston 4 can be observed in the viewing window 1h and the plunger is in the most proximal position for a full syringe. The advancement of the injection button 21 activates the delivery mechanism enclosed in the proximal part of the housing 1 thereby advancing the piston 4 in the prefilled syringe 2 such that medicament can be expelled via the needle 6 (Figure 1d). Once the prefilled syringe has been emptied, the user may release the injection button 21 which is biased by the delivery mechanism to move back from the button actuated position 23 to the button retracted position 22 over the button actuation distance 24 (Figure 1e). The delivery mechanism biases the prefilled syringe 2 to move from the needle inserted position to the needle retracted position as the injection button and the prefilled syringe are operatively coupled. The injection device 32 may optionally include a lock out mechanism locking the injection button in the needle retracted position preventing re-use of the device and ensures that the needle of the prefilled syringe is covered within the distal end of the housing to prevent needle stick injuries (Figure 1e).

The injection may be paused during the injection (e.g. when the prefilled syringe has not been completely emptied yet) by releasing injection button 21 to an intermediate position between the button actuated position and the button retracted position. Medicament delivery is temporarily stalled until the user pushes the injection button again to move into the button actuated position and resuming medicament delivery. The pausing of the medicament delivery may be repeated until the prefilled syringe has been emptied as a lockout mechanism for the injection button will not be activated. Pausing of the medicament delivery may be initiated by a user sensing pain during an injection of, for example, a high viscous liquid medicament or when injecting cold fluids. Details for the pausing function will be described further below.

The injection may be terminated during the injection (e.g. when the prefilled syringe has not been completely emptied) by completely releasing the injection button which is moved back by the delivery mechanism to the button retracted position and the lockout mechanism is activated such that the user cannot move the injection button again towards the button actuated position. The lockout mechanism is not activated during pausing of the injection as the injection button is moved to an intermediate position and not completely back to the button retracted position. Details for the lock out mechanism will be described further below.

### Parts of the injection device:

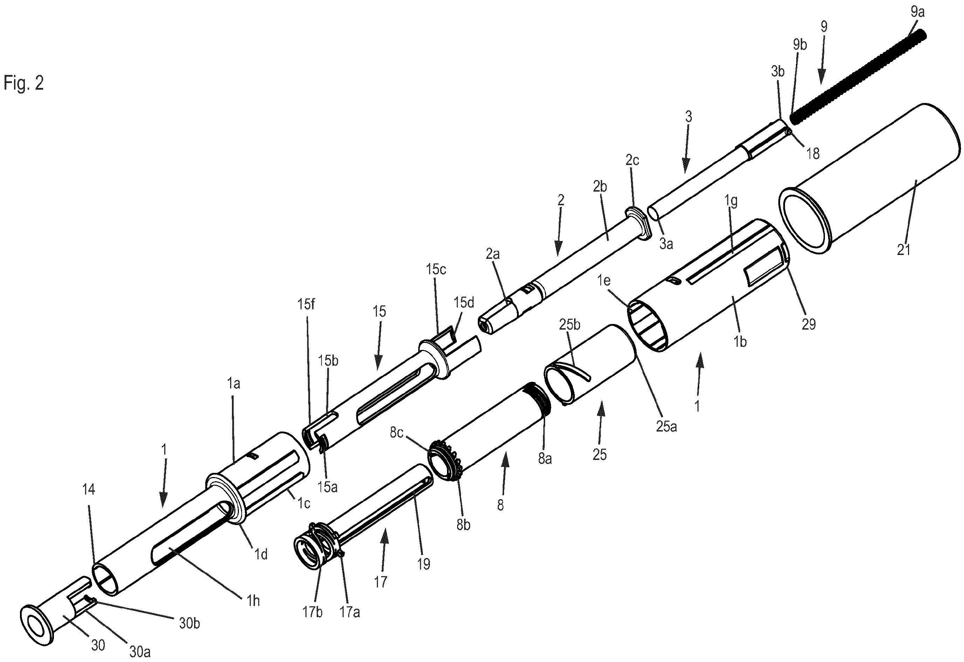

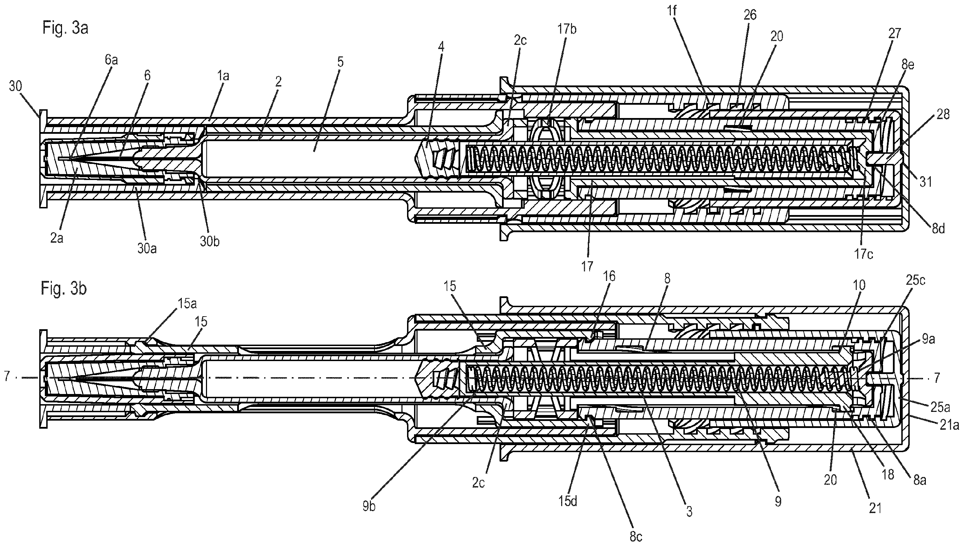

An exploded view for the auto injector 32 is presented in Figure 2. The auto injector 32 includes a housing 1 provided as two housing parts that may be connected to each other. Functionally and structurally the two housing parts behave as a housing 1 facilitating the assembly of the auto injector device 32. A distal housing 1a comprises a sleeve shaped proximal section 1j coupled to a sleeve shaped distal end section 1k of different outer dimension that includes the viewing window 1h. The distal end 14 for the distal housing 1a is configured for receiving a needle shield remover 30. The distal housing 1a is configured to receive and axially guide a syringe holder 15. The distal housing 1a includes longitudinal keys 1c on the outside surface that are adapted to be received by longitudinal guide slots 1e on the inside surface of a proximal housing 1b such that the proximal housing 1b is guided during assembly with the distal housing 1a. The distal housing 1a includes a rim 1d providing an axial stop for the movement of the proximal housing 1b during assembly. The proximal housing 1b and the distal housing 1a may be snap-fitted together by engagement of a protrusion 11 on the proximal sleeve 1j engaging an opening 1m in the wall of the proximal housing 1b thereby providing a snap fit connector 1n (Figure 3a) for an assembled housing 1.

The syringe holder 15 is axially guided and moveable with respect to the housing 1, the syringe holder may be, for example keyed to the distal housing 1a. The syringe holder 15 includes a sleeve shaped body configured to receive a prefilled syringe 2. The syringe holder 15 includes a viewing window 15e and two extensions 15f extend axially from the distal end of the syringe holder 15 leaving two cut outs 15b configured to receive two arms 30a of the needle shield remover 30. The needle shield remover 30 is rotationally coupled to the syringe holder 15 by the engagement of the arms 15f and 30a. Two arms 15c extend from the proximal end of the syringe holder 15 and are configured to receive and rotationally secure a finger flange 2c of the prefilled syringe 2. The syringe holder 15 holds and supports the prefilled syringe 2 when moving the syringe holder 15 with respect to the housing 1. The prefilled syringe 2 includes a glass barrel 2b with a stacked needle on the distal end and closed by a plunger on the proximal end defining a volume available for the medicament. The prefilled syringe 2 is closed at the distal end by a needle shield 2a. The needle shield 2a may be a rigid or a soft needle shield. The arms 30a of the needle shield remover 30 that fit into the cut outs 15b of the syringe holder 15 may extend over the needle shield 2a and rims 30b may protrude radially inward from the arms 30a of the needle shield remover 30 for engaging the needle shield 2a.

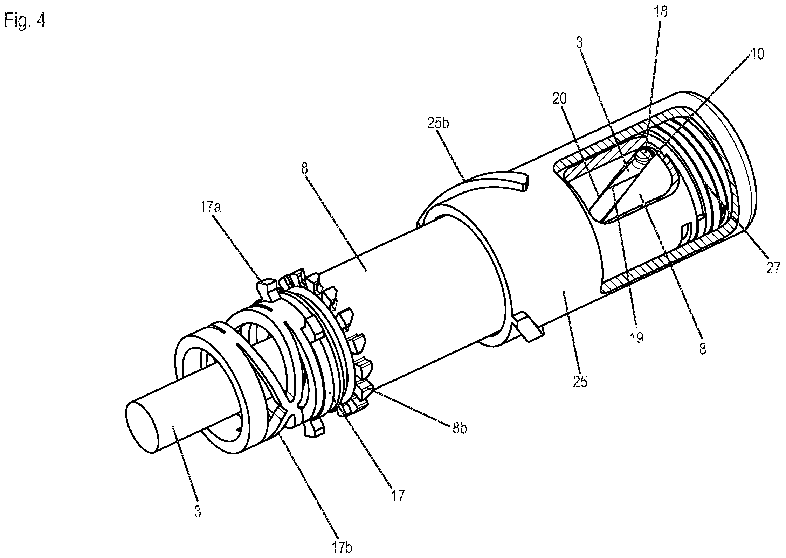

A plunger rod 3 includes a distal end 3a that is configured to be inserted in the prefilled syringe 2 and abut a plunger 4 as shown in Figure 3a. The plunger rod 3 is provided as a hollow sleeve enclosing an injection spring 9 and the distal end 9b of the injection spring 9 may abut a distal end wall of the plunger rod 3 for transferring the load from the injection spring 9 to the plunger rod 3. The proximal end 9a of the injection spring 9 abuts an end wall of a spring sleeve 19 and the end wall of the spring sleeve may engage a clutch sleeve that will be presented below. The spring sleeve 17 coaxially surrounds the plunger rod 3 and the release sleeve 8 coaxially surrounds the spring sleeve 17. The spring force in the proximal direction may be transferred from the injection spring 9 to the release sleeve 8 via the spring sleeve 17. The force may be transferred to the release sleeve via the clutch sleeve as will be presented below. The release sleeve 8 includes a circumferential recess 8c at the distal end that is configured to engage the rim 15d located at the extensions 15c of the syringe holder. The engagement 8c,15d may provide an axial coupling between the syringe holder 2 and the release sleeve 8 whereas the release sleeve 8 may rotate relative to the syringe holder 2. The spring sleeve 17 includes a spring element 17b at its distal end which is configured to bias the prefilled syringe 2 in the distal direction into the syringe holder 15. The spring sleeve includes radial protrusions 17a (Figure 2) configured to engage axial guide slots (not shown) on the inside surface of the distal housing 1a such that the spring sleeve 17 is axially guided and rotationally coupled to the housing 1. The spring sleeve 17 includes at least one guide slot 19 oriented parallel to the longitudinal axis 7 configured to engage at least one protrusion 18 extending radially outwards from the proximal end 3b of the plunger rod 3. The engagement between the protrusion 18 and the guide slot 19, respectively between the protrusions 8b and the housing provide for a rotational coupling between the plunger rod 3 and the housing 1. The protrusion 18 on the plunger rod 3 further engages a helical groove or first thread segment 10 (Figure 3b) on an inside surface of the release sleeve 8 providing a first threaded engagement 10 that will be presented in more detail in Figure 4. The bias provided by the injection spring 9 on the plunger rod 3 may result in a rotational torque on the release sleeve 8 via the first threaded engagement 10. The rotational torque may be guided to the housing by a releasable coupling between the release sleeve 8 and the housing 1. Release of the coupling between the housing 1 and the release sleeve 8 may start the injection process thereby advancing the plunger rod 3 and rotating the release sleeve 8.

Needle insertion mechanism: The proximal housing 1b includes an axial groove 1g on the outside surface (Figures 2 and 3b) for engaging and axially guiding protrusions 21b on the inside surface of the injection button 21 (Figure 3b). The end of the axial grooves 1g and/or the proximal end 29 of the proximal housing 1b may provide stops for the axial movement of the injection button 21 and may include stops preventing removing the injection button from the proximal end 29 of the proximal housing 1b (Figure 3b). The injection button 21, or an end wall of the injection button abuts a proximal end wall 25a of a clutch sleeve 25 such that a distal movement of the injection button 21 may be transmitted to a distal movement of the clutch sleeve 25. The clutch sleeve 25 includes a thread segment 25b extending radially outward from the outside surface of the clutch sleeve 25 configured to engage a helical groove 1f (Figure 3a) on the inside of the proximal housing 1b thereby providing a third threaded engagement which is non-self-locking. The clutch sleeve 25 is configured to rotate as the injection button is moved towards the button actuated position. The clutch sleeve 25 includes an internal thread segment or groove 25c (Figure 3b) adapted to engage an external thread segment 8a located at the proximal end of the release sleeve 8 thereby providing a second threaded engagement. The release sleeve 8 is configured to axially move together with the clutch sleeve 25 due to the second threaded engagement. The clutch sleeve 25 rotates around the non-rotating release sleeve 8 as the injection button 21 is moved towards the button actuated position. The release sleeve 8 is axially coupled to the syringe holder 15 and the syringe holder 15, including the prefilled syringe, is adapted to be moved by the injection button 21 via the clutch sleeve 25 from the needle retracted position towards the needle inserted position.

### Assembled auto injector in the initial state:

Longitudinal sections for the assembled auto injector 32 in the initial state are presented in Figures 3a and 3b. The two sections are taken in two planes oriented perpendicular to each other. The needle shield remover 30 is attached to the distal end of the housing 1. The arms 30a extend in the proximal direction and the rims 30b protruding from the proximal end of the arms 30a engage a proximal rim of the needle shield 2a. The needle shield remover 30 is axially guided by the housing and the arms 30a engage the cut-outs 15b of the syringe holder 15 thereby preventing rotation of the needle shield remover 30. A protrusion 15a or a protruding rim is located at the distal end of the syringe holder 15 engaging a complementary recess 1o on the distal end of the housing 1a to from a releasable snap-fit connected. The presence of the needle shield 30 prevents the release of the snap-fit connection and therewith axial movement between the housing 1 and the syringe holder 15 along the longitudinal axis 7.

The prefilled syringe 2 is provided in the syringe holder 15 and a liquid medicament 5 may be present between the needle 6 and a piston 4. The flange 2c is biased into the distal direction by the spring element 17b. The syringe holder 15 is axially coupled to the release sleeve 8 by coupling 16 provided by rims 15d engaging circumferential recess 8c. The release sleeve 8 includes a helical groove 20 on an inside surface adapted to engage the protrusion 18 of the plunger rod 3 thereby providing the first threaded engagement 10 that will be explained in more detail in Figure 4. The spring sleeve 17 surrounds the plunger rod 3 and the release sleeve 8 surrounds the spring sleeve 17. The injection spring 9 is located within the sleeve shaped plunger rod 3. The distal end 9b of the injection spring 9 abuts an end wall 3a of the plunger rod 3 and the proximal end 9a abuts the end wall 17c of the spring sleeve. The end wall 8e of the release sleeve 8 includes a passage 8d configured to receive a protrusion 28 that protrudes from the end wall 25a of the clutch sleeve 25. The injection spring 9 biases the plunger rod 3 into the distal direction and biases the spring sleeve 17 and the clutch sleeve 25 (via the protrusion 28) into the proximal direction. The clutch sleeve 25 is engaged with the release sleeve 8 via the second threaded engagement 27 provided by the thread segment 8a on the outside surface of the release sleeve 8 and an internal helical groove 25c on the inside surface of the clutch sleeve 25. The bias in the proximal direction is transferred from the injection spring to the spring sleeve, from the spring sleeve to the clutch sleeve and from the clutch sleeve to the release sleeve (via the second threaded engagement). The clutch sleeve 25 is engaged with the proximal housing 1b via the third threaded engagement 26 provided by a helical rib or thread segment 25b on the outside surface of the clutch sleeve 25 and a helical groove 1f on an inner surface of the proximal housing 1b. The injection button 21 surrounds the cutch sleeve 25 and is axially guided by the proximal housing 1b. A bearing, for example a pivot bearing 31, may be provided between the proximal end wall 21a of the injection button and the proximal end wall 25a of the clutch sleeve 25 to reduce frictional forces between a non-rotating injection button 21 and a rotating clutch sleeve 25 upon device activation. An axial force provided on the injection button 21 is directly transferred to the clutch sleeve 25 via the mutual engagement of the end walls and/or the pivot bearing 31. The axial force provided on the injection button 21 is transferred to the spring sleeve 17 via the protrusion 28 of the clutch sleeve 28 and from the clutch sleeve 25 to the release sleeve 8 via the second threaded engagement 27. The syringe holder 15 is axially coupled to the release sleeve 8 and the syringe holder is prevented from axially moving relative to the housing 1 due to the presence of the needle shield remover 30 as described above. In the initial state of the auto injection device 32, the assembly of the syringe holder 15, the release sleeve 8, the spring sleeve 17, the clutch sleeve 25 and the injection button 21 is prevented from axial movement towards the distal end of the housing by the presence of the needle shield remover 30 thereby preventing device activation.

A perspective view for an assembly showing a part of the release mechanism is depicted in Figure 4. The plunger rod 3 is surrounded by the spring sleeve 17. The spring sleeve 17 is axially guided by the housing using protrusions 17a engaging longitudinal grooves on the inside surface of the housing 1. The plunger rod 3 is axially guided by the protrusion 18 engaging the longitudinal slot 19 of the spring sleeve 17. The release sleeve 8 is releasably coupled to the housing by protrusions 8b engaging longitudinal slots on the inside surface of the housing 1. The protrusion 18 on the plunger rod 3 engages the helical groove 20 on the inside surface of the release sleeve 8 thereby providing the first threaded engagement 10. The injection spring 9 located inside the plunger rod 3 intends to move the plunger rod 3 in the distal direction and provides a torque on the release sleeve 8 via the first threaded engagement 10. The release sleeve 8 is rotationally coupled to the housing via protrusions 8b thereby preventing rotation of the release sleeve and advancement of the plunger rod 3. Axial movement of the release sleeve 8 via the second threaded engagement 27 with the clutch sleeve 25 may decouple the release sleeve from the housing 1 thereby allowing rotation of the release sleeve and advancement of the plunger rod 3.

### Removing the needle shield remover:

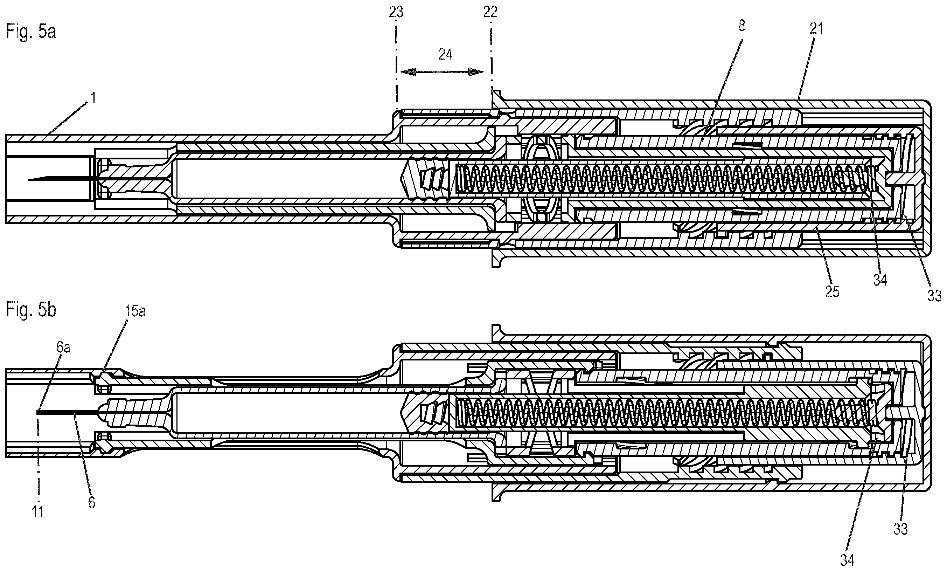

Longitudinal sections for the assembled auto injector 32 in a state after removing the needle shield remover 30 are presented in Figures 5a and 5b, and the sections are taken in two planes oriented perpendicular to another. Removing the needle shield remover 30 simultaneously removes the needle shield 2a as the protruding rim 30a of the needle shield remover 30 engages the proximal end of the needle shield 2a during distal movement of the needle shield remover 30. The tip 6a of the needle is covered by the distal end of the housing and is in the needle retracted position 11. The snap fit connection between the protrusion 15a on the syringe holder and the recess on the housing may be released by axially moving the syringe holder against a threshold force when pressing the injection button 21 and moving the button from the button retracted position 22 towards the button actuated position 23.

### Moving the injection button:

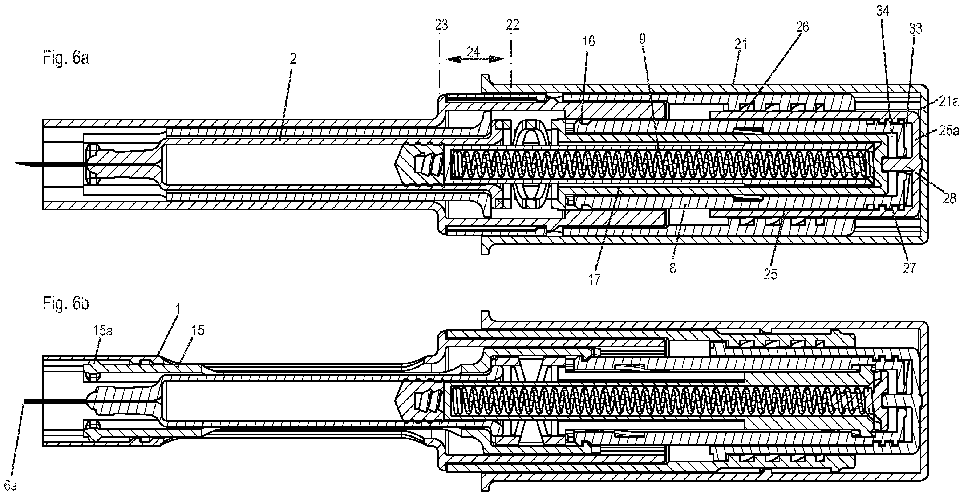

Longitudinal sections for the assembled auto injector 32 in a state with the injection button moved from the button retracted position 22 to a position between the button retracted position 22 and the button actuated position 23 are presented in Figures 6a and 6b. The snap-fit connection between the housing and the syringe holder has been released either by local elastic or plastic deformation of the housing and/or elastic deformation of the syringe holder 15. The injection button 21 is axially moved without rotation and the axial load is transferred via the end wall 21a to the end wall 25a of the clutch sleeve 25. The clutch sleeve axially advances and starts rotating due the third threaded engagement 26 between the clutch sleeve 25 and the proximal housing 1b. The release sleeve 8 is axially advanced towards the distal end of the housing 1 due to the second threaded engagement 27. The clutch sleeve 25 rotates around the non-rotating release sleeve 8 as the release sleeve remains rotationally coupled to the housing when the protrusions 8b slide trough the complementary guide slots on the inside surface of the housing. The axial distance between the clutch sleeve 25 and the release sleeve 8, defined as a first axial gap 33, is reduced due to the relative rotation between the clutch sleeve 25 and the release sleeve 8. The release sleeve 8 is axially moved towards end wall 25a of the clutch sleeve 25 and the release sleeve 8 draws the syringe holder 15 (and prefilled syringe 2) towards the clutch sleeve 25 as the release sleeve 8 is axially coupled to the syringe holder via coupling 16. The axial distance between the spring sleeve 17 and the piston rod 3 is reduced as well indicated as second axial gap 34 (compare Figures 5a and 6a). The second and third threaded engagement provide a gearing arrangement gearing down the axial movement of the injection button against the proximal directed spring force provided by the injection spring 9 that is directed via the spring sleeve 17 to the clutch sleeve 25 (using protrusion 28) and finally to the injection button 21. Closing the second axial gap 34 compresses the injection spring 9 and the gearing arrangement supports to gear down the force required for that compression. The syringe holder 15 and therewith the prefilled syringe is moved from the needle retracted position 11 (Figure 5b) towards the needle inserted position and the tip of the needle 6a projects from the distal end of the housing 1 (Figure 6b).

### Injection button moved to the actuated position:

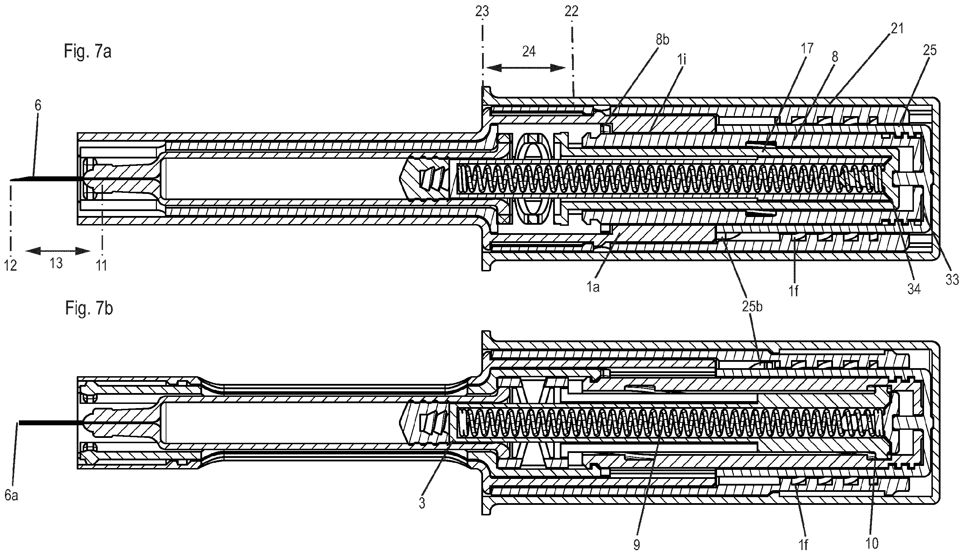

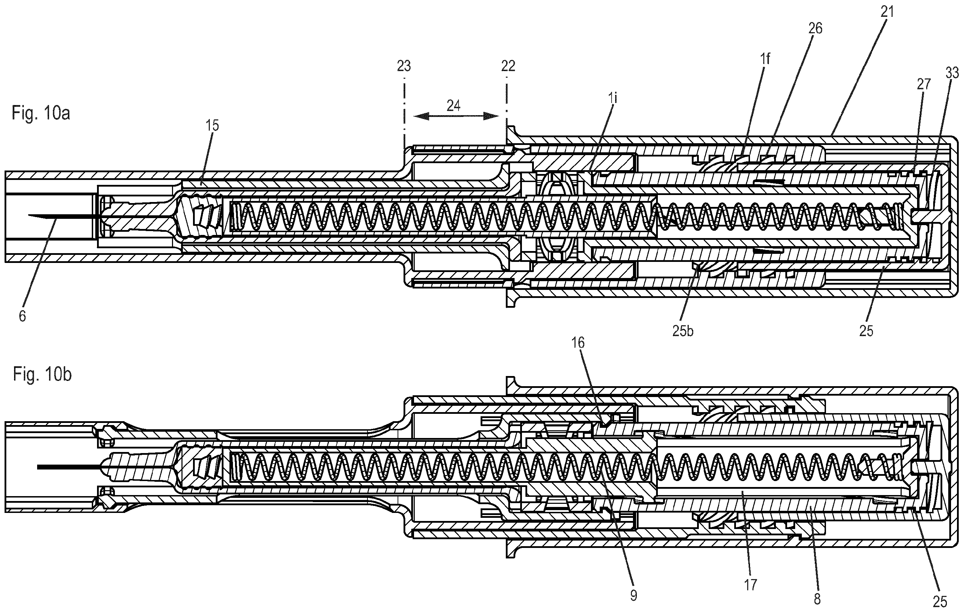

Longitudinal sections for the assembled auto injector 32 in a state with the injection button moved into the button actuated position 23 are presented in Figures 7a and 7b, respectively. The injection button has moved into the button actuated position 23. The syringe holder 15 and the prefilled syringe 2 have moved to the needle inserted position 12 and the tip 6a of the needle 6 fully extends from the distal end of the housing. The clutch sleeve 25 has been rotated and axially advanced and the first axial gap 33 between the clutch sleeve 25 and the release sleeve 8 has been closed. The injection button 21 has been moved over the button actuation distance 24 and the prefilled syringe has been moved over the needle insertion distance 13 (Figure 7a). The needle insertion distance 13 can be calculated as the button actuation 24 reduced by the initial distance for the first axial gap 33 (Figure 5a). The needle insertion distance may be, for example, 16 mm and the first axial gap 2 mm. The needle insertion distance amounts in this case 14 mm. The second axial gap 34 has been closed and the injection spring 9 is fully compressed by, in the example presented before, an additional 2 mm. The additional compression of the injection spring 9 is required to initiate retraction of the injection needle 6 and retraction of the injection button 21 as will be described below using Figures 10a and 10b. The release sleeve 8 has axially moved towards the distal end of the housing and the protrusions 8b at the distal end of the release sleeve are disengaged from guide slots 1i on the inside of the distal housing 1a. The release sleeve 8 is rotationally decoupled from the housing 1. The third threaded engagement 26 between the thread segment 25b on the outside surface of the clutch sleeve 25 and the helical groove 1f on the inside surface of the proximal housing 1b has been released as well. The clutch sleeve 25 has been rotated over an angle around the longitudinal axis such that the thread segment 25b is disengaged from groove 1f. Either the release sleeve 8 is first decoupled from the housing followed by releasing the third threaded engagement or the third threaded engagement 26 between the clutch sleeve 25 and the housing 1 is released first followed by decoupling the release sleeve 8 from the housing. Alternatively both are decoupled simultaneously. The plunger rod 3 will advance towards the distal end of the housing due to the bias provided by the injection spring 9 and torque applied to the release sleeve 8 via the first threaded engagement 10 that will rotate the release sleeve 8. The clutch sleeve 25 co-rotates with the release sleeve due to the second threaded engagement. The clutch sleeve can free rotate without axial movement as the third threaded 26 engagement is disengaged.

### Plunger rod advancement:

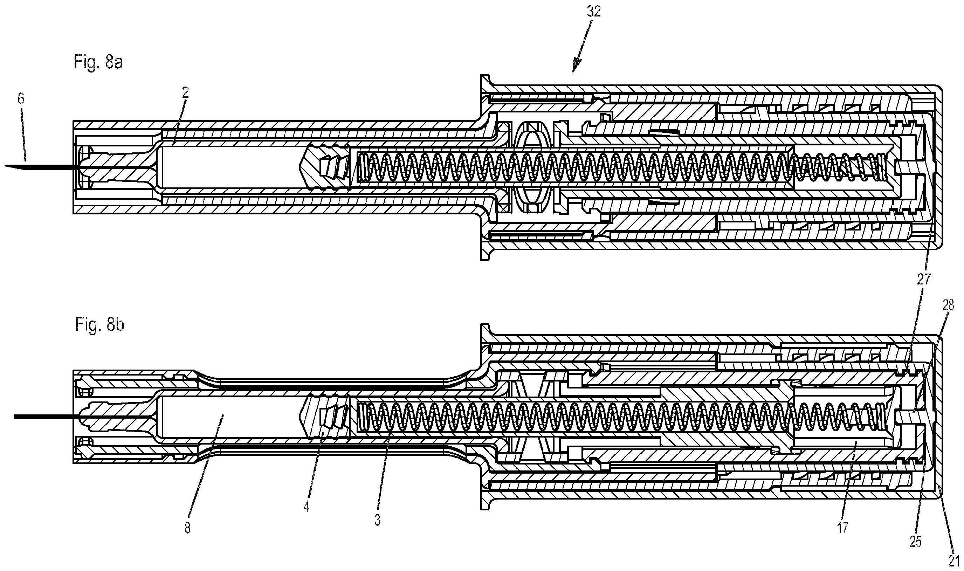

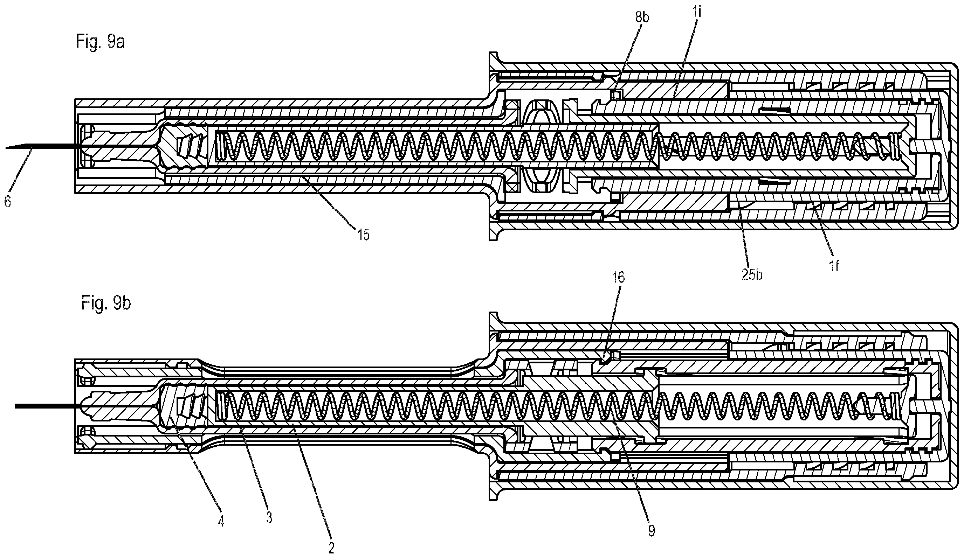

Longitudinal sections for the assembled auto injector 32 in a state where the plunger rod has moved to an intermediate position are presented in Figures 8a and 8b. The needle 6 is in the inserted position and the plunger rod 4 has advanced the plunger 4 towards the distal end of the prefilled syringe thereby expelling medicament 5 through the hollow needle 6. The injection spring 9 is at least partially decompressed. The user pushes the auto injector 32 against the reactive force provided by the injection spring pushing the spring sleeve 17, the clutch sleeve 25 (via protrusion 28), the release sleeve 8 (via the second threaded engagement 27), and the injection button 21 in the proximal direction. Finally, the plunger rod 3 has moved the plunger 4 to the most distal position as presented in Figures 9a and 9b. All medicament available has been delivered from the auto injection device in a single shot. During delivery, the release sleeve 8 rotates versus the non-rotating syringe holder 15 as allowed by the coupling 16.

### Needle retraction:

Longitudinal sections for the assembled auto injector 32 in a state where injection button 21 has moved back to the button retracted position after medicament delivery are presented in Figures 10a and 10b, respectively. The user releases the injection button 21 and the injection spring 9 pushes the assembly of the spring sleeve 17, the clutch sleeve 25, the release sleeve 8, and via the axial coupling 16, the syringe holder 15 in the proximal direction. The needle 6 is retracted into the distal end of the housing 1 for reducing the risk of needle sticking as the device is removed from the skin. The coupling between protrusions 8b and grooves 1i (see Figure 9a) is reinstalled as the release sleeve 8 moves into the proximal direction. The protrusions 1i and/or the grooves 1i may have facetted or rounded entrance sections facilitating correct closure of the coupling. The thread segment 25b and the helical grooves 1f on the housing re-engage as the clutch sleeve 25 is moved in the proximal direction. The gearing engagement using the second and third threaded engagements 27, 26 ensures that the first axial gap 33 between the clutch sleeve 25 and the release sleeve 8 is opened as the clutch sleeve 25 rotates around the non-rotating release sleeve 8 which has been rotationally coupled to the housing. The clutch sleeve 25 is axially moved in the proximal direction while rotating around the release sleeve 8. The additional spring force built up during needle insertion is used to move the assembly including the injection button 21 towards the proximal direction. The release sleeve 8, the syringe holder 15, the spring sleeve 17 and the syringe are moved back from the needle inserted position to the needle retracted position over the needle insertion distance whereas the clutch sleeve 25 and the injection button are moved from the button actuated position to the button retracted position over the button actuation distance.

### Lock out mechanism:

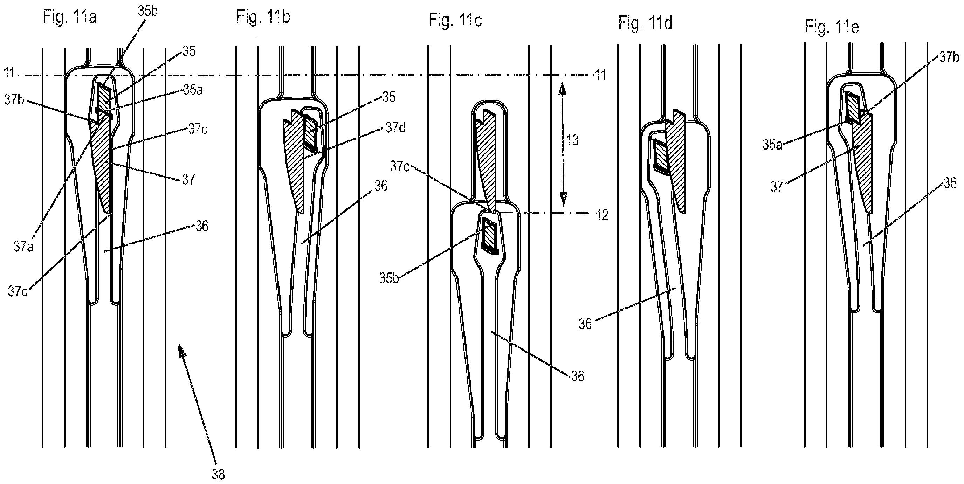

In one aspect of the invention the auto injection device may include a lock out mechanism 38 preventing re-use once the injection button has been completely moved back from the button actuated position to the button retracted position. The lockout mechanism 38 is presented in Figures 11a to 11e. The lockout mechanism includes a protrusion 35 on a flexible arm 36 on one component of the auto injection device that is moved relative to another component of the auto injection device that includes a guiding protrusion 37. The protrusion 35 and flexible arm 36 may be provided on the syringe holder 15, the spring sleeve 17 or the injection button 21. The guiding protrusion may be provided on the housing 1. The lockout mechanism may be activated when the syringe holder 15 or the spring sleeve 17 is moved back over the needle insertion distance from the needle inserted position completely back to the needle retracted position. Movement to an intermediate position will be described below for pausing the injection. The lockout mechanism may be activated when the injection button 21 is moved back over the button actuation distance from the button actuated position completely back to the button retracted position. Movement to an intermediate position will described below for pausing the injection. As a non-limiting example, the lockout mechanism between the syringe holder 15 and the distal housing 1a will be explained in the following.

The guiding protrusion 37 is provided on the distal housing 1a and the flexible arm 36 with the protrusion 35 on the syringe holder 15. The protrusion 35 is located at the proximal end of the flexible arm 36. In the initial state of the auto injection device (see Figures 3a and 3b), a first facet 35a on the protrusion 35 abuts a complementary facet 37a on the guiding protrusion 37 on the housing (Figure 11a). The functional sequence of the lock out mechanism is described in the following. The syringe holder 15 is moved from the needle retracted position 11 towards the distal end of the housing during needle insertion (see Figures 6a and 6b) and the first facet 35a slides over the complementary facet 37a such that the protrusion 35 can slide along the side wall 37d of the guiding protrusion 37 on the housing thereby flexing the flexible arm 36, see Figure 11b. The syringe holder is moved into the needle inserted position 12 (see Figures 7a and 7b) and the flexible arm 36 flexes back to a neutral position as the protrusion 35 passes beyond the distal end 37c of the guiding protrusion 37 on the housing (Figure 11c). During the needle retraction, for example after completing the injection or during pausing of an injection, the syringe holder 17 moves in the proximal direction and a second facet 35b on the protrusion 35 of the syringe holder engages the tip 37c on the guiding protrusion 37 such that the arm 36 flexes in the opposite direction (compare Figures 11b and 11d). The lockout mechanism as presented in Figure 11d is not in a locked position yet and the syringe holder may be moved again in the distal direction by pushing the injection button 21 again. If the syringe holder is moved back to its utmost proximal position (the needle retracted position of the injection needle 6) then the arm 36 flexes back and the first facet 35a of the protrusion on the syringe holder is biased by the flexible arm 36 into a locking engagement with a cut out 37b on the guiding protrusion 37 of the housing (Figure 11e). The syringe holder cannot be moved towards the distal end of the housing as the lockout mechanism is in a locked position preventing re-use of the auto injection device and retaining the injection needle within the housing.

### Pausing the injection:

The injection may be temporarily paused if the user may sense pain during an injection. The injection has been started but the prefilled syringe 2 has not been emptied yet, the release sleeve 8 is rotating as the plunger rod 3 moves in the distal direction, this is the situation depicted in Figure 8a for the position of the plunger rod and Figure 11c for the lockout mechanism. The user may release the injection button 21 such that the injection button moves towards an intermediate position between the button actuated position and the button retracted position such that the release sleeve 8 is rotationally coupled to the housing thereby halting the advancement of the plunger rod. The lockout mechanism is not locked yet (Figure 11d) and after pausing the injection, the user may push the injection button 21 again towards the button actuated position such that the release sleeve 8 is decoupled from housing and is allowed to rotate for advancement of the plunger rod 3. The lockout mechanism is again in the position depicted in Figure 11c. After emptying the prefilled syringe, the user releases the injection button that moves back to the button retracted position and the lockout mechanism moves in a locking mode (Figure 11e)

### Delivery of a partial dose:

A partial dose may be delivered from the injection device. The plunger rod has not been moved to the final position (Figure 8a) and the user releases the injection button such that the lockout mechanism enters into the locking mode. The prefilled syringe has not been emptied but the injection cannot be started again.

**PART ANNOTATION**

| | | | |
|---|---|---|---|
| 1 | Housing | 9a | Proximal end |
| 1a | Distal housing | 9b | Distal end |
| 1b | Proximal housing | 10 | First threaded engagement |
| 1c | Longitudinal key | 11 | Needle retracted position |
| 1d | Rim | 12 | Needle inserted position |
| 1e | Longitudinal slot | 13 | Needle insertion distance |
| 1f | Helical groove | 14 | Distal end housing |
| 1g | Longitudinal groove | 15 | Syringe holder |
| 1h | Viewing window | 15a | Protrusion |
| 1i | Guide slot | 15b | Cut out |
| 1j | Proximal section | 15c | Arms |
| 1k | Distal section | 15d | Rim |
| 1l | Protrusion | 15e | Viewing window |
| 1m | Opening | 15f | Extension |
| 1n | Snap-fit connection | 16 | Axial coupling |
| 1o | Recess | 17 | Spring sleeve |
| 2 | Prefilled syringe | 17a | Radial protrusion |
| 2a | Needle shield | 17b | Spring element |
| 2b | Glass barrel | 17c | End wall |
| 2c | Flange | 18 | Protrusion |
| 3 | Plunger rod | 19 | Guide slot |
| 3a | Distal end | 20 | First thread segment |
| 3b | Proximal end | 21 | Injection button, injection |
| 4 | Piston, Plunger | | sleeve |
| 5 | Medicament | 21a | Proximal end wall |
| 6 | Needle, cannula | 21b | Protrusion |
| 6a | Needle tip | 22 | Button retracted position |
| 7 | Longitudinal axis | 23 | Button actuated position |
| 8 | Release sleeve | 24 | Button actuation distance |
| 8a | Thread segment | 25 | Clutch sleeve |
| 8b | Radial protrusions | 25a | Proximal end wall |
| 8c | Circumferential recess | 25b | Thread segment |
| 8d | Passage, opening | 25c | Helical groove |
| 8e | End wall | 26 | Third threaded engagement |
| 9 | Injection spring | 27 | Second threaded engagement |
| 28 | Protrusion | 35a | First facet |
| 29 | Proximal end housing | 35b | Second facet |
| 30 | Needle shield remover, cap | 36 | Flexible arm |
| 30a | Arms | 37 | Guiding protrusion |
| 30b | Protruding rim | 37a | Facet guiding protrusion |
| 31 | Pivot bearing | 37b | Cut-out / Stop surface |
| 32 | Auto injector | 37c | Tip |
| 33 | First axial gap | 37d | Side wall |
| 34 | Second axial gap | 38 | Lockout mechanism |
| 35 | Protrusion on flexible arm | | |

## Claims

1. An assembly for an injection device (32) comprising
a housing (1) defining a longitudinal axis,
a syringe (2) with an attached needle (6), the syringe (2) being axially moveable with respect to the housing (1) along the longitudinal axis between a needle retracted position (11) and a needle inserted position (12) defining a needle insertion distance (13),
a plunger rod (3), axially moveable and rotationally secured to the housing (1) and configured to engage a piston (4) in the syringe (2) for expelling medicament from the injection device,
a release sleeve (8) axially moveable relative to the housing (1),
an injection spring (9) operatively arranged between the plunger rod (3) and the release sleeve (8) whereby the injection spring (9) is configured to bias the plunger rod (3) towards the distal end (14) of the housing,
a first threaded engagement (10) between the release sleeve (8) and the plunger rod (3) configured to rotate the release sleeve (8) when the plunger rod (3) moves towards the distal end of the housing,
wherein the release sleeve (8) is rotationally coupled to the housing (1) when the prefilled syringe (2) is axially moved from the needle retracted position (11) towards the needle inserted position (12),
wherein the release sleeve (8) is rotationally decoupled from the housing (1) when the prefilled syringe (2) has reached the needle inserted position (12) thereby allowing the injection spring (9) to move the plunger rod (3) towards the distal end of the housing while rotating the release sleeve (8) via the first threaded engagement (10).

2. The assembly according to claim 1, further comprising a syringe holder (15) for holding the syringe (2), the syringe holder (15) being axially moveable relative to the housing (1) over the needle insertion distance (13).

3. The assembly according to claim 2 wherein the syringe holder (15) is axially coupled to the release sleeve (8) and the release sleeve is configured to be rotatable relative to the syringe holder.

4. The assembly according to claims 1 to 3 further comprising a spring sleeve (17) that is rotationally coupled to the housing (1) and axially moveable relative to the housing (1) over the needle insertion distance (13).

5. The assembly according to claim 4, wherein the spring sleeve (17) is coaxially arranged between the plunger rod (3) and the release sleeve (8).

6. The assembly according to claims 4 or 5 wherein the plunger rod (3) comprises at least one protrusion (18) oriented perpendicular to the longitudinal axis engaging a guide slot (19) in the spring sleeve (17), the guide slot (19) oriented parallel to the longitudinal axis, thereby axially guiding the plunger rod (3).

7. The assembly according to claim 6 wherein the at least one protrusion (18) engages a first thread segment (20) on the inside of the release sleeve (8), the first thread segment (20) having a first pitch whereby the first thread segment (20) and the protrusion (18) provide the first threaded engagement (10).

8. The assembly according to any of the previous claims further comprising an injection button (21) axially guided by the housing (1) and moveable between a button retracted position (22) and a button actuated position (23) defining a button actuation distance (24).

9. The assembly according to claim 8 wherein the button actuation distance (24) is greater than the needle insertion distance (13).

10. The assembly according to claim 9 wherein a clutch sleeve (25)is coaxially arranged between the injection button (21) and the release sleeve (8).

11. The assembly according to claim 10 wherein the clutch sleeve (25) comprises a proximal end wall (25a) abutting the injection button (21).

12. The assembly according to claim 11 wherein the clutch sleeve (25) is threadedly engaged with the housing (1) via a third threaded engagement (26) such that the clutch sleeve (25) rotates when the dose button (21) axially moves from the button retracted position (22) to the button actuated position (23).

13. The assembly according to claim 12 wherein the clutch sleeve (25) is threadedly engaged with the release sleeve (8) via a second threaded engagement (27) and wherein the second and third threaded engagements have the same hand.

14. The assembly according to claim 13 wherein the clutch sleeve (25) comprises a protrusion (28) oriented along the longitudinal axis and guided through an opening (8d) in the release sleeve (8) and wherein the protrusion (8d) abuts a distal end of the spring sleeve (17).

15. The assembly according to claims 12 to 14 wherein the clutch sleeve (25) rotates around the release sleeve (8) as the injection button (21) axially moves towards the button actuated position (23) and wherein the release sleeve (8) axially moves towards the clutch sleeve(25) due to the second and third threaded engagements (27, 26) thereby compressing the injection spring (9) by a distance equal to the difference between the button actuation distance (24) and the needle insertion distance (13).
